# EUROPEAN PATENT APPLICATION

(11) **EP 4 741 000 A1**
(43) Date of publication of application: **13.05.2026**
(21) Application number: 25214226.0
(22) Date of filing: 07.11.2025
(51) Int. Cl.: A61N 1/39, A61H 31/00

(54) **GUIDANCE DEVICE FOR DETECTING CARDIOPULMONARY RESUSCITATION PERFORMANCE**

(30) Priority: 08.11.2024 KR 20240158108; 31.12.2024 KR 20240202678
(71) Applicant: CU Medical Systems Inc., Wonju-si, Gangwon-do 26365 (KR)
(72) Inventor: NOH, Tae Jong, 26439 Wonju-si (KR)
(74) Representative: Schiweck Weinzierl Koch Patentanwälte Partnerschaft mbB

(57) **Abstract**

A guidance device through detection of cardiopulmonary resuscitation (CPR) performance includes a pair of electrode pads configured to be attached to a patient's body; a signal providing unit configured to provide a signal to the pair of electrode pads; a differential amplifier connected to the pair of electrode pads and configured to generate an amplified signal proportional to a difference between input signals at both ends provided from the signal providing unit; and a control unit configured to store a normal speed range for a plurality of amplified signals generated from the differential amplifier, and determine whether CPR is being performed and whether CPR is performed normally based on a speed of the plurality of amplified signals generated from the differential amplifier.

## Description

### CROSS-REFERENCE TO RELATED APPLICATIONS

This application claims the priority of Korean Patent Application No. 10-2024-0158108 filed on November 8, 2024, in the Korean Intellectual Property Office, the disclosure of which is incorporated herein by reference.

### BACKGROUND

### Field

The present disclosure relates to a guidance device through detection of cardiopulmonary resuscitation (CPR) performance, and more particularly, to a guidance device through detection of CPR performance that provides guidance voice for CPR.

### Description of the Related Art

Rapid cardiopulmonary resuscitation (CPR) and defibrillation performed on a patient with cardiac arrest may increase the patient's survival rate. Automated defibrillators that can be easily used by not only medical personnel but also non-medical personnel are installed in public places such as multi-use facilities, schools, and public institutions.

Korean Registered Patent No. 10-1049273 entitled "Automatic Defibrillator and Its Operating Method" includes a voice guidance unit for guiding treatment steps for a patient by voice to a rescuer, a step execution evaluation unit for evaluating an execution degree for each step of the guidance voice of the voice guidance unit, and a guidance voice change unit for changing the guidance voice step of the voice guidance unit according to an evaluation result of the step execution evaluation unit. This patent evaluates the rescuer's level of using the defibrillator and provides customized voice guidance according to the user's level. International Patent Application Publication No. WO 2005/099818 A2 entitled "Automated Pediatric Defibrillator" includes a user interface having a graphical display unit and a text display unit provided on a cover, and a display unit or a voice speaker that provides prompts to the rescuer to assist in performing treatment for the patient. It is guided to output a voice or text prompt to the rescuer such that the rescuer may recognize the patient's condition and perform defibrillation.

However, the conventional rescue guidance devices provide guidance for performing defibrillation, but do not provide any guidance on performing CPR by professional or non-professional rescuers after the defibrillation is performed. In particular, since non-professional rescuers have no experience or training in CPR, they may perform chest compressions at a speed that is too slow or too fast in urgent situations. In addition, although professional rescuers are skilled in emergency treatments such as CPR, they are hindered from providing quicker rescue due to excessive and uniform guidance provided to all users.

### SUMMARY

An object to be achieved by the present disclosure is to provide a guidance device through detection of cardiopulmonary resuscitation (CPR) performance, which may determine whether CPR is being performed and whether the CPR is performed normally, and provide guidance accordingly.

Another object of the present disclosure is to provide a guidance device through detection of cardiopulmonary resuscitation (CPR) performance, which may improve both efficiency and accuracy of CPR performance by providing CPR guidance suitable for both skilled and unskilled users. In conventional defibrillation devices, CPR guidance is provided according to voice guidance set at the time of product release. However, when the voice guidance is set in a detailed manner, it may interfere with fast and prompt CPR performance by skilled users, and when the voice guidance is set in a simplified manner, it may delay CPR performance by unskilled users. In order to solve these problems, the present disclosure monitors changes in thoracic impedance between both ends of defibrillation pads for a predetermined period of time from the point when CPR voice guidance begins, and is designed such that when CPR is normally performed, the CPR voice guidance proceeds in a simplified manner.

A guidance device through detection of cardiopulmonary resuscitation (CPR) performance according to the present disclosure for achieving the above object includes: a pair of electrode pads configured to be attached to a patient's body; a signal providing unit configured to provide a signal to the pair of electrode pads; a differential amplifier connected to the pair of electrode pads and configured to generate an amplified signal proportional to a difference between input signals at both ends provided from the signal providing unit; and a control unit configured to store a normal speed range for a plurality of amplified signals generated from the differential amplifier, and determine whether CPR is being performed and whether CPR is performed normally based on the plurality of amplified signals generated from the differential amplifier and a speed of the plurality of amplified signals generated from the differential amplifier. When a pair of electrode pads is attached to a chest area of a patient, whether CPR is being performed may be determined based on an amplitude difference of a plurality of amplified signals generated from a differential amplifier, and then whether CPR is performed normally may be determined according to whether a speed of the amplified signals of the differential amplifier is within a normal speed, so that voice guidance may be provided accordingly.

Here, the guidance device may further include a speaker configured to output guidance voice for CPR, and the control unit may store guidance voice regarding a CPR performing method and control the speaker so that the guidance voice regarding the CPR performing method is output when an amplified signal generated from the differential amplifier is received. In particular, since the CPR technique is guided to a non-professional rescuer, even a non-professional rescuer who is not a professional rescuer may easily perform CPR, which is preferable.

In addition, the control unit may store guidance voice regarding whether CPR is being performed and whether CPR is performed normally, and control the speaker so that the corresponding guidance voice is output when it is determined, based on the plurality of amplified signals generated from the differential amplifier, that CPR is not being performed. If there is no difference in input signals from the pair of electrode pads, it is recognized that CPR is not being performed, so that guidance voice regarding CPR performance and a CPR performing method may be output, which is preferable.

Here, the control unit may store a simplified guidance voice and a detailed guidance voice regarding whether CPR is performed normally, and control output of the simplified guidance voice when a speed of the plurality of amplified signals generated from the differential amplifier is within the normal speed range, and control output of the detailed guidance voice when the speed of the plurality of amplified signals generated from the differential amplifier deviates from the normal speed range. Accordingly, when CPR is performed normally, the simplified guidance voice in the form of a beep sound may be output according to a set chest compression rate (100 to 120 times per minute), and when CPR is performed abnormally, the detailed guidance voice regarding chest compressions being too slow or too fast may be output, which is preferable.

The control unit may store a threshold number of times for CPR, calculate the number of performances of CPR based on the plurality of amplified signals generated from the differential amplifier, determine whether the calculated number of performances exceeds the threshold number of times, and control output of any one of the simplified guidance voice and the detailed guidance voice according to the determination result. Accordingly, when the number of performances does not exceed the threshold number of times, the simplified guidance voice in the form of a beep sound corresponding to a set chest compression rate (100 to 120 times per minute) may be output so that chest compressions continue, and when the number of performances exceeds the threshold number of times, the detailed guidance voice may be output so that further chest compressions are no longer performed, which is preferable.

Here, the control unit may monitor, in real time, a state and performing accuracy of a rescuer during CPR performance, and output the detailed guidance voice step by step according to basic stages, intermediate processes, and special situations of CPR.

In addition, the control unit may provide basic instruction on a chest compression position, compression depth, and compression rate in a basic stage of CPR, evaluate the compression depth and rate in real time in an intermediate process and provide feedback when they deviate from a normal range, and provide the detailed guidance voice configured to provide immediate instructions regarding fatigue of a rescuer, inaccuracy of hand position, or inconsistency of compression rhythm in a special situation.

Here, when a pressure sensor for measuring, in real time, pressure generated during CPR is attached to a chest compression area of CPR, the control unit may calculate compression depth and rate based on data collected through the pressure sensor, and convert the calculated result into voice and visual feedback.

In addition, the pressure sensor may be configured to be one selected from a piezoresistive pressure sensor, a piezoelectric sensor, or a capacitive pressure sensor, and the control unit may determine whether CPR is being performed and whether CPR is performed normally based on pressure data detected from the pressure sensor.

Here, the control unit may provide, based on data collected from the pressure sensor, immediate guidance to a rescuer through voice and visual guidance via a speaker and a display unit when a compression depth is insufficient or excessive, or when a compression rate deviates from a normal range.

The guidance method through detection of cardiopulmonary resuscitation (CPR) performance according to the present disclosure for achieving the above object includes: storing a normal speed range for a plurality of amplified signals generated from a differential amplifier that is connected to a pair of electrode pads attached to a patient's body and configured to generate the amplified signals proportional to a difference between input signals at both ends provided from a signal providing unit; and determining, based on a speed of the plurality of amplified signals generated from the differential amplifier, whether CPR is being performed and whether the CPR is performed normally. When the pair of electrode pads is attached to a chest area of a patient, whether CPR is being performed may be determined based on an amplitude difference of the plurality of amplified signals generated from the differential amplifier, and then whether the CPR is performed normally may be determined according to whether a speed of the amplified signals of the differential amplifier is within a normal speed, so that voice guidance may be provided accordingly.

Here, the method includes: storing guidance voice regarding a CPR performing method; and outputting the guidance voice regarding the CPR performing method when an amplified signal generated from the differential amplifier is received. In particular, the CPR technique may be guided to a non-professional rescuer, so that even a non-professional rescuer who is not a professional rescuer may easily perform CPR, which is preferable.

In addition, the method may further include: storing guidance voice regarding whether CPR is being performed and whether CPR is performed normally; and outputting the guidance voice when it is determined, based on a speed of the plurality of amplified signals generated from the differential amplifier, that CPR is not being performed. Thus, when there is no difference in input signals from the pair of electrode pads, it is recognized that CPR is not being performed, so that guidance voice regarding CPR performance and a CPR performing method may be output, which is preferable.

Here, the method may further include: storing a simplified guidance voice and a detailed guidance voice regarding whether CPR is performed normally; outputting the simplified guidance voice when a speed of the plurality of amplified signals generated from the differential amplifier is within the normal speed range; and outputting the detailed guidance voice when the speed of the plurality of amplified signals generated from the differential amplifier deviates from the normal speed range. Thus, when CPR is performed normally, the simplified guidance voice in the form of a beep sound may be output according to a set chest compression rate (100 to 120 times per minute), and when CPR is performed abnormally, the detailed guidance voice regarding chest compressions being too slow or too fast may be output, which is preferable.

In addition, the method may further include: storing a threshold number of times for CPR; calculating the number of performances of CPR based on the plurality of amplified signals generated from the differential amplifier; determining whether the calculated number of performances exceeds the threshold number of times; and outputting any one of the simplified guidance voice and the detailed guidance voice according to the determination result. Accordingly, when the number of performances does not exceed the threshold number of times, the simplified guidance voice in the form of a beep sound corresponding to a set chest compression rate (100 to 120 times per minute) may be output so that chest compressions continue, and when the number of performances exceeds the threshold number of times, the detailed guidance voice may be output so that further chest compressions are no longer performed, which is preferable.

According to the present disclosure, when a pair of electrode pads is attached to the chest of a patient, whether cardiopulmonary resuscitation (CPR) is being performed may be determined based on the amplitude difference of a plurality of amplified signals generated from a differential amplifier, and then whether the CPR is performed normally may be determined according to whether the speed of the amplified signal of the differential amplifier is within a normal speed, thereby providing voice guidance accordingly.

In addition, since the CPR technique is guided to a non-professional rescuer, even a non-professional rescuer who is not a professional rescuer may easily perform CPR.

In addition, if there is no difference in input signals from the pair of electrode pads, it is recognized that CPR is not being performed, so that guidance voice regarding the CPR performance and a CPR performing method may be output.

In addition, when CPR is performed normally, a simplified guidance voice in the form of a beep sound corresponding to the set chest compression rate (100 to 120 times per minute) may be output, and when CPR is performed abnormally, a detailed guidance voice regarding chest compressions being too slow or too fast may be output.

In addition, when the number of performances does not exceed a threshold number of times, a simplified guidance voice in the form of a beep sound corresponding to the set chest compression rate (100 to 120 times per minute) may be output so that chest compressions continue, and when the number of performances exceeds the threshold number of times, a detailed guidance voice may be output so that chest compressions are no longer performed.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIG. 1 is an exemplary diagram of a guidance device through detection of cardiopulmonary resuscitation performance according to the present disclosure;
FIG. 2 is an exemplary diagram of an output signal for cardiopulmonary resuscitation;
FIG. 3 is an exemplary diagram of an output signal for a chest compression rate;
FIG. 4 is a control block diagram of a guidance device through detection of cardiopulmonary resuscitation performance;
FIG. 5 is an overall operation diagram of a guidance method through detection of cardiopulmonary resuscitation performance;
FIG. 6 is a flowchart of a first exemplary embodiment of a guidance method through detection of cardiopulmonary resuscitation performance according to the present disclosure;
FIG. 7 is a flowchart of a second exemplary embodiment of a guidance method through detection of cardiopulmonary resuscitation performance;
FIG. 8 is a flowchart of a third exemplary embodiment of a guidance method through detection of cardiopulmonary resuscitation performance;
FIG. 9 is a flowchart of a fourth exemplary embodiment of a guidance method through detection of cardiopulmonary resuscitation performance; and
FIG. 10 is a flowchart of a fifth exemplary embodiment of a guidance method through detection of cardiopulmonary resuscitation performance.

### DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

Hereinafter, a guidance device through detection of cardiopulmonary resuscitation (CPR) performance 1 according to a preferred embodiment of the present disclosure will be described in detail with reference to the accompanying drawings.

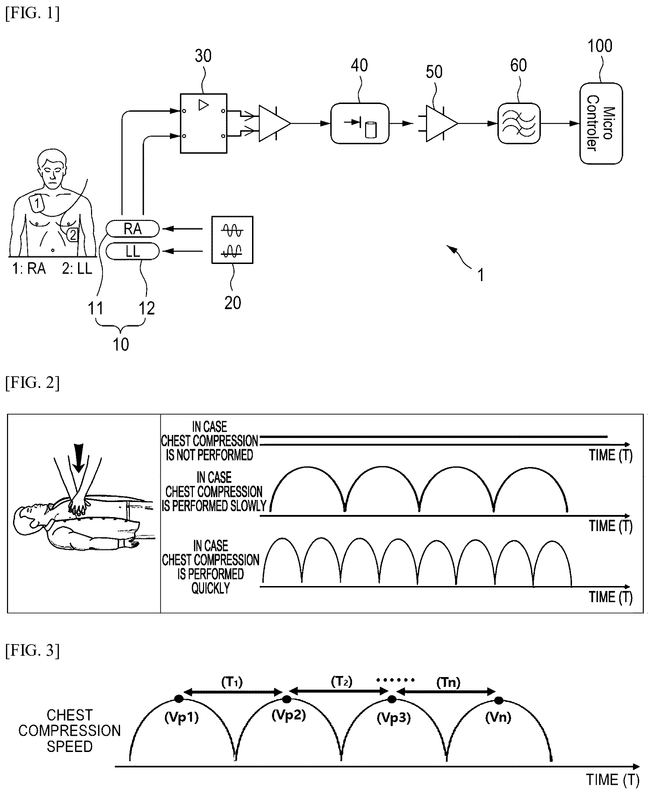

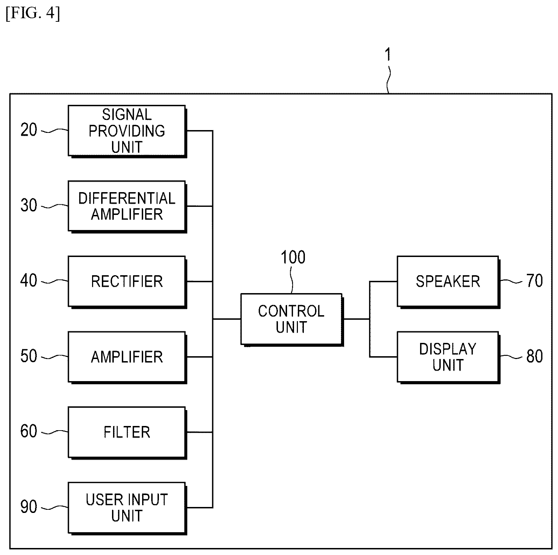

FIG. 1 is an exemplary diagram of a guidance device through detection of cardiopulmonary resuscitation performance 1 according to the present disclosure. FIG. 2 is an exemplary diagram of an output signal related to cardiopulmonary resuscitation. FIG. 3 is an exemplary diagram of an output signal with respect to a chest compression rate. FIG. 4 is a control block diagram of a guidance device through detection of cardiopulmonary resuscitation performance 1.

Referring to FIGS. 1 to 4, the configuration of the guidance device through detection of cardiopulmonary resuscitation (CPR) performance 1 will be described.

The guidance device through detection of cardiopulmonary resuscitation (CPR) performance 1 includes electrode pads 10, a signal providing unit 20, a differential amplifier 30, a rectifier 40, an amplifier 50, a filter 60, a speaker 70, a display unit 80, a user input unit 90, and a control unit 100.

The electrode pads 10 are a pair of components attached to the patient's body. The electrode pads 10 include a first electrode pad 11 and a second electrode pad 12.

The first electrode pad 11 is attached to an area of the patient's chest and transmits a signal varied by chest compression provided from the signal providing unit 20 to the differential amplifier 30.

The second electrode pad 12 is attached to another area of the patient's chest, opposite to the area where the first electrode pad 11 is attached with respect to the heart of the patient, and transmits a signal varied by chest compression provided from the signal providing unit 20 to the differential amplifier 30.

The signal providing unit 20 provides a signal of constant strength to the pair of electrode pads 10.

The differential amplifier 30 is connected to the pair of electrode pads 10 and generates an amplified signal proportional to a difference between the input signals at both ends provided by the signal providing unit 20. The input signals input from the pair of electrode pads 10 vary in amplitude according to the chest compression of the rescuer, and this is amplified.

The rectifier 40 converts alternating current (AC), which periodically changes direction, into direct current (DC) that flows in only one direction.

The amplifier 50 amplifies the direct current signal converted by the rectifier 40.

The filter 60 filters the signal amplified by the amplifier 50 to remove noise and transmits it to the control unit 100.

The speaker 70 may output guidance voice related to cardiopulmonary resuscitation (CPR).

The display unit 80 displays an image based on an image signal processed by image processing. The implementation method of the display unit 80 is not limited, but may be implemented by various display methods such as liquid crystal, plasma, light emitting diode, organic light emitting diode, surface-conduction electron-emitter, carbon nano-tube, or nano-crystal.

The display unit 80 may additionally include auxiliary configurations depending on its implementation method. For example, when the display unit 80 is a liquid crystal type, the display unit 80 may include a liquid crystal display panel (not shown), a backlight unit (not shown) for supplying light thereto, and a panel driving board (not shown) for driving the panel (not shown). The display unit 80 may display a speech recognition result as information on recognized voice. Here, the speech recognition result may be displayed in various forms such as text, graphics, and icons, and the text includes letters and numbers. The display unit 80 may further display candidate commands and application information according to the speech recognition result. The user may check whether the voice is correctly recognized through the speech recognition result displayed on the display unit 80, and may operate the user input unit 90 provided in a remote controller to select a command corresponding to the voice uttered by the user among the displayed candidate commands, or to select and confirm information related to the speech recognition result.

The user input unit 90 may be implemented as an input means by which a user may input a user command. The user input unit 90 may receive a user's touch input or remote input using a remote controller and transmit it to the control unit 100. In addition, the user input unit 90 may receive a voice input uttered by the user and transmit the voice signal to the control unit 100. In such a case, the user input unit 90 may be implemented, for example, as a microphone. The user input unit 90 may also perform signal processing on the received voice signal by itself. However, the type of user input that may be received by the user input unit 90 is not limited thereto, and may also include, for example, user input received through motion recognition.

The control unit 100 may store the normal speed range for a plurality of amplified signals generated by the differential amplifier 30, and determine whether CPR is being performed and whether CPR is being normally performed, based on amplitude differences of the plurality of amplified signals generated by the differential amplifier 30 and on speeds of the plurality of amplified signals generated by the differential amplifier 30.

The control unit 100 may store guidance voice related to a CPR performing method, and when an amplified signal generated by the differential amplifier 30 is received, it may control the speaker 70 to output the guidance voice related to the CPR performing method.

The control unit 100 may store guidance voice related to whether CPR is being performed and whether CPR is being normally performed, and when it determines, based on a plurality of amplified signals generated by the differential amplifier 30, that CPR is not being performed, it may control the corresponding guidance voice to be output.

The control unit 100 may store a simplified guidance voice and a detailed guidance voice related to whether CPR is being normally performed, and may control the simplified guidance voice to be output when the speed of a plurality of amplified signals generated by the differential amplifier 30 is within the normal speed range, and may control the detailed guidance voice to be output when the speed of a plurality of amplified signals generated by the differential amplifier 30 deviates from the normal speed range.

The control unit 100 may store a threshold number of times for CPR, calculate the number of CPR performances based on a plurality of amplified signals generated by the differential amplifier 30, determine whether the calculated number of CPR performances exceeds the threshold number of times, and control any one of the simplified guidance voice and the detailed guidance voice to be output according to the determination result.

The control unit 100 may monitor the rescuer's condition and performance accuracy in real time during CPR, and may output the detailed guidance voice step by step according to basic stages, intermediate processes, and special situations of CPR.

The control unit 100 may provide the detailed guidance voice configured to provide basic instructions regarding chest compression position, compression depth, and compression speed in the basic stage of CPR, evaluate compression depth and speed in real time during the intermediate process, and provide feedback when they deviate from the normal range, and provide immediate instructions in special situations regarding rescuer fatigue, incorrect hand position, or compression rhythm mismatch.

The control unit 100 may attach a pressure sensor to a chest compression area of CPR to measure, in real time, pressure generated during CPR, calculate compression depth and speed based on data collected through the pressure sensor, and convert the calculated result into voice and visual feedback.

Here, the pressure sensor may be configured by selecting one of a piezoresistive pressure sensor, a piezoelectric sensor, or a capacitive pressure sensor.

The control unit 100 may determine whether CPR is being performed and whether CPR is being normally performed, based on pressure data detected by the pressure sensor.

Based on data collected from the pressure sensor, the control unit 100 may immediately provide the rescuer with voice and visual guidance through the speaker and the display unit when compression depth is insufficient or excessive, or when compression speed deviates from the normal range.

In the guidance device through detection of cardiopulmonary resuscitation (CPR) performance 1 according to the present disclosure, the simplified guidance voice provides a simple metronome tone so that the rescuer may maintain a set chest compression speed and effectively perform CPR. A simple signal tone, such as a "beep" sound, is repeatedly output so that the rescuer may maintain rhythm according to the set chest compression speed (100 to 120 times per minute). The simplified guidance voice is configured simply to maintain compression rhythm without additional explanations or detailed instructions.

Meanwhile, the detailed guidance voice is designed to be provided step by step by monitoring the rescuer's condition and performance accuracy in real time during CPR. This system is intended to provide optimal feedback to the rescuer during the basic stage, the intermediate process, and special situations of CPR. In the basic stage, basic instructions at the start of CPR are provided to the rescuer. For example, guidance on compression position, such as "Press the central area between both nipples, the lower one-third of the sternum," is provided together with compression depth guidance, such as "For adults, press more than about 5 cm, and for children, press about 5 cm," and compression speed guidance, such as "Maintain a speed of about 100 to 120 times per minute." Such basic guidance is composed simply and clearly so that even a beginner may easily understand and support the basic stage of CPR.

During the intermediate stage in which CPR is being performed, the rescuer's chest compression speed and depth are monitored in real time, and immediate feedback is provided when they deviate from the guideline. For example, when the compression speed is too fast, guidance such as "The chest compression speed is too fast. Adjust to 120 times per minute or less" is provided, and when the speed is too slow, feedback such as "The chest compression speed is too slow. Maintain at least 100 times per minute" is output. In addition, when the compression depth is insufficient or excessive, guidance such as "The compression depth is insufficient. Press the chest at least 5 cm" or "The compression depth is too deep. Adjust the compression appropriately" is provided, respectively. Since the chest may not be fully released during CPR, an additional voice such as "After chest compression, fully release the chest so that the heart is filled with blood" is output. Such real-time feedback helps the rescuer continue to perform CPR correctly.

Since CPR is a high-intensity task, there is a possibility that quality may deteriorate due to rescuer fatigue. In order to prevent this, the detailed guidance voice provides instructions for fatigue management at regular intervals. Messages such as "Switch rescuers every two minutes" or "If you feel fatigue, immediately switch with another rescuer to maintain compression quality" are output so that the rescuer may prevent quality deterioration caused by excessive fatigue. The compression depth and speed may be checked in real time through the AED display, and guidance such as "The compression depth is insufficient. Press deeper" or "The compression depth is too deep. Adjust the compression" is also provided.

In order to determine the appropriateness of compression depth, a pressure sensor is installed on the chest compression area of CPR to measure, in real time, pressure generated during CPR. The pressure sensor may be selected from a piezoresistive pressure sensor, a piezoelectric sensor, or a capacitive pressure sensor. The piezoresistive pressure sensor detects pressure through resistance change according to pressure, the piezoelectric sensor generates an electrical signal by pressure, and the capacitive pressure sensor converts capacitance change according to pressure into a signal. Such sensors must have a measurement range of 0 to 100 mmHg and a fast response speed, and must be suitable for the general pressure range of CPR.

When a pressure sensor is installed on the chest compression area, the arrangement of the sensor must consider accuracy and durability. When the sensor is attached to the center of the chest compression area, the accuracy of pressure data may be ensured, and it must be designed with a flexible structure so that the sensor does not interfere with CPR. In addition, an appropriate shielding layer must be introduced to prevent interference between the sensor and surrounding electrical systems, and it is necessary to minimize signal interference problems. The data collected from the pressure sensor must be integrated into the differential amplifier and the control unit. The differential amplifier amplifies the pressure data to prevent signal loss, and the control unit analyzes the data to evaluate CPR quality in real time and provide appropriate feedback to the rescuer.

The pressure sensor serves as a core component of the guidance device through detection of cardiopulmonary resuscitation (CPR) performance according to the present disclosure, and may operate by being connected to the differential amplifier and the control unit. The sensor attached to the chest compression area detects pressure in real time and converts it into an electrical signal, the differential amplifier amplifies the signal, and the rectifier and the filter process it. The control unit analyzes the processed signal to calculate compression depth and speed, and may determine whether CPR is being normally performed. According to the analysis result, voice and visual feedback may be provided to the rescuer through the speaker and the display unit. The pressure sensor is linked with the electrode pads to monitor CPR quality in real time and provide feedback, thereby enhancing the effectiveness of CPR.

Even in special situations, the detailed guidance voice provides immediate feedback. For example, since the position of the hands may be incorrect, guidance such as "Press with the heel of your palm. Do not let your fingers touch the chest" is output, and when the compression position deviates, feedback such as "Adjust the compression position to the central area between both nipples" is provided. Along with this, when there is a problem with the compression rhythm, instructions such as "Maintain a constant rhythm. Perform chest compression according to the metronome tone" are output to maximize the effectiveness of CPR.

Since children and infants have structural characteristics different from adults, differentiated guidance for them is also included in the detailed guidance voice. For children, guidance such as "For children, press the center of the sternum with one hand or two fingers. Maintain a depth of about 5 cm" is provided, and for infants, detailed instructions such as "For infants, use two fingers to press the center of the sternum at a depth of about 4 cm. Maintain a speed of 100 to 120 times per minute" are output. This is customized guidance to ensure safety and effective CPR for children and infants.

Finally, the AED display and voice guidance are integrated to help monitor CPR quality visually and auditorily. Messages such as "Check the AED screen to maintain compression speed and depth" or "The compression speed is out of the proper range. Adjust according to the guidance" are provided so that the rescuer may immediately check and improve the quality of their CPR performance.

In conclusion, the detailed guidance voice provides basic instructions in the basic stage of CPR, maintains quality in the intermediate stage through real-time feedback, and provides guidance for immediate problem solving in special situations. This supports the rescuer in performing CPR correctly and maximizes the efficiency and accuracy of CPR.

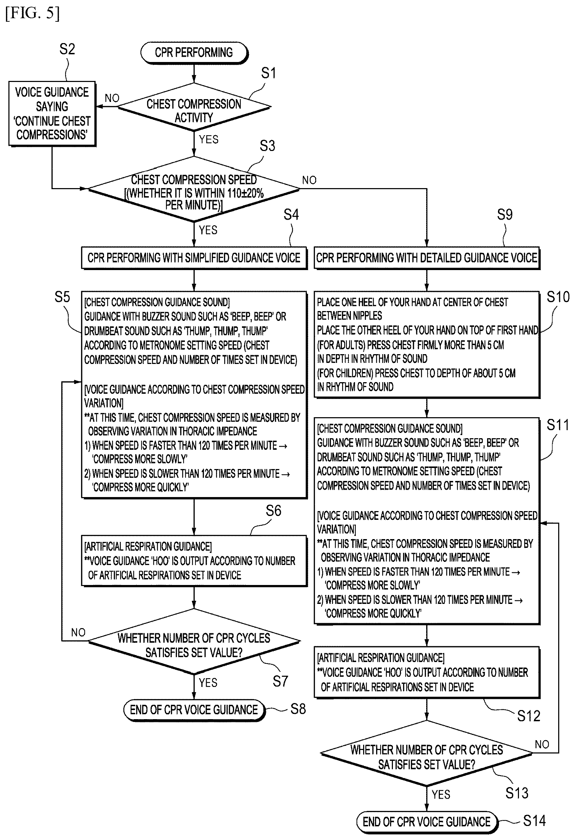

FIG. 5 is an overall operation diagram of a guidance method through detection of cardiopulmonary resuscitation (CPR) performance.

The rescuer performs CPR while applying chest compressions to the patient (S1).

When the control unit 100 determines that there is no amplitude difference in a plurality of amplified signals generated by the differential amplifier 30, it determines that the rescuer is not performing chest compressions and outputs a guidance voice stating, "Continue chest compressions" (S2).

It is determined whether the chest compression speed of the rescuer is within the normal speed range of 110 ± 20% per minute (S3).

When the rescuer's chest compression speed is within the normal speed range, a simplified guidance voice is output (S4). Here, the simplified guidance voice may be a beep sound corresponding to the chest compression speed (100 to 120 times per minute), and since the rescuer may perform chest compressions according to this beep sound, chest compressions may be performed at the correct speed.

While the rescuer continues chest compressions, when the chest compression speed exceeds 120 times per minute, which is the normal speed range, a detailed guidance voice such as "slower" is output, and when the chest compression speed falls short of 120 times per minute, a detailed guidance voice such as "faster" is output (S5).

A preset voice guidance for artificial respiration is output (S6).

It is determined whether the number of CPR performances exceeds a threshold number of times (S7).

When the number of CPR performances exceeds the threshold number of times, a guidance voice to stop CPR is output (S8).

Here, when the number of CPR performances does not exceed the threshold number of times, step S5 continues to be performed.

When, in step S3, the rescuer's chest compression speed is not within the normal speed range, a detailed guidance voice is output (S9).

Here, by outputting a detailed guidance voice for CPR, it is possible to inform a non-professional rescuer of the CPR performing method (S10). Here, step S10 may also be performed before step S1, in which CPR is carried out.

While the non-professional rescuer continues chest compressions, when the chest compression speed exceeds 120 times per minute, which is the normal speed range, a detailed guidance voice such as "slower" is output, and when the chest compression speed falls short of 120 times per minute, a detailed guidance voice such as "faster" is output (S11).

A preset voice guidance for artificial respiration is output (S12).

It is determined whether the number of CPR performances exceeds a threshold number of times (S13).

When the number of CPR performances exceeds the threshold number of times, a guidance voice to stop CPR is output (S14).

Here, when the number of CPR performances does not exceed the threshold number of times, step S11 continues to be performed.

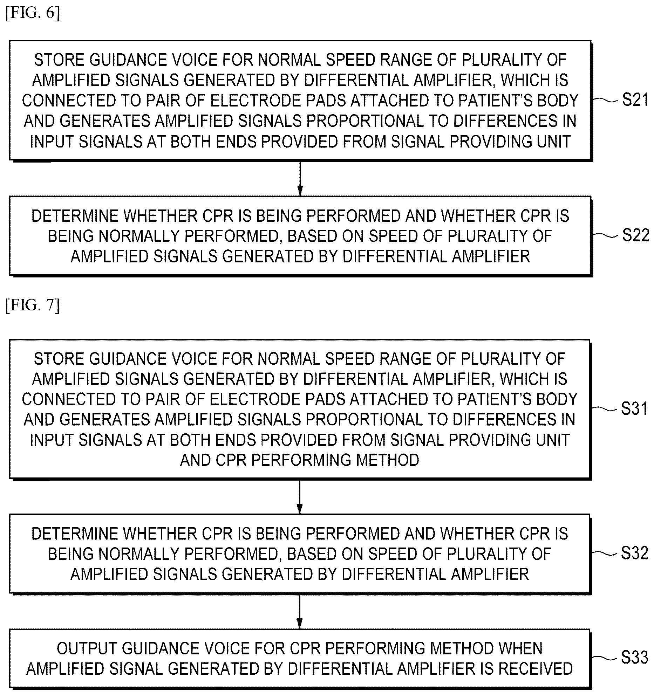

FIG. 6 is a flowchart of a first exemplary embodiment of a guidance method through detection of cardiopulmonary resuscitation (CPR) performance according to the present disclosure.

The control unit 100 is connected to a pair of electrode pads 11 and 12 attached to the patient's body and stores guidance voice for a normal speed range of a plurality of amplified signals generated by the differential amplifier 30, which generates amplified signals proportional to differences in input signals at both ends provided from the signal providing unit 20 (S21).

The control unit 100 determines whether CPR is being performed and whether CPR is being normally performed, based on the speed of a plurality of amplified signals generated by the differential amplifier 30 (S22).

FIG. 7 is a flowchart of a second exemplary embodiment of a guidance method through detection of cardiopulmonary resuscitation (CPR) performance.

The control unit 100 is connected to a pair of electrode pads 11 and 12 attached to the patient's body and stores guidance voice related to a normal speed range of a plurality of amplified signals generated by the differential amplifier 30, which generates amplified signals proportional to differences in input signals at both ends provided from the signal providing unit 20, and a CPR performing method (S31).

The control unit 100 determines whether CPR is being performed and whether CPR is being normally performed, based on the speed of a plurality of amplified signals generated by the differential amplifier 30 (S32).

When an amplified signal generated by the differential amplifier 30 is received, the control unit 100 outputs guidance voice for a CPR performing method (S33).

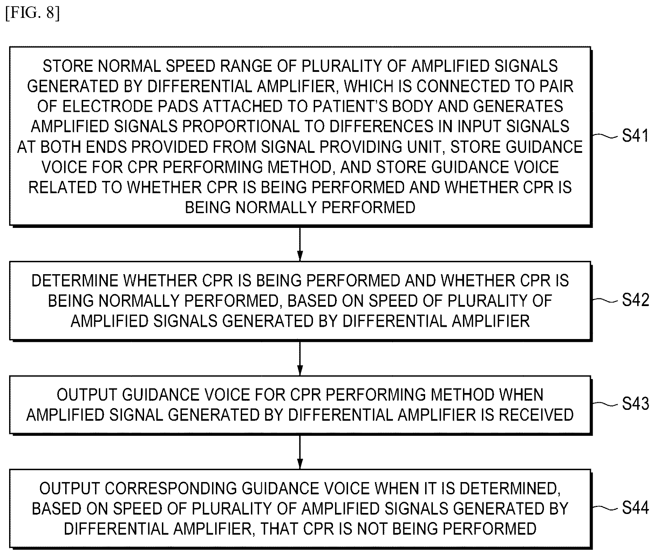

FIG. 8 is a flowchart of a third exemplary embodiment of a guidance method through detection of cardiopulmonary resuscitation (CPR) performance.

The control unit 100 is connected to a pair of electrode pads 11, 12 attached to the patient's body and stores guidance voice related to a normal speed range of a plurality of amplified signals generated by the differential amplifier 30, which generates amplified signals proportional to differences in input signals at both ends provided from the signal providing unit 20, stores guidance voice for a CPR performing method, and stores guidance voice related to whether CPR is being performed and whether CPR is being normally performed (S41).

The control unit 100 determines whether CPR is being performed and whether CPR is being normally performed, based on the speed of a plurality of amplified signals generated by the differential amplifier 30 (S42).

When an amplified signal generated by the differential amplifier 30 is received, the control unit 100 outputs guidance voice for a CPR performing method (S43).

The control unit 100 may output the corresponding guidance voice when it determines, based on the speed of a plurality of amplified signals generated by the differential amplifier 30, that CPR is not being performed (S44).

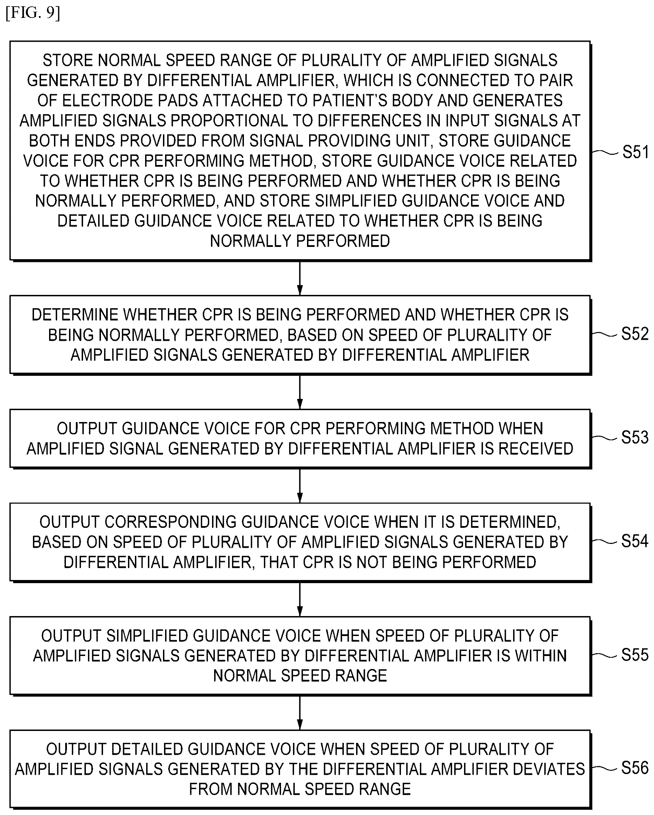

FIG. 9 is a flowchart of a fourth exemplary embodiment of a guidance method through detection of cardiopulmonary resuscitation (CPR) performance.

The control unit 100 is connected to a pair of electrode pads 11, 12 attached to the patient's body and stores guidance voice related to a normal speed range of a plurality of amplified signals generated by the differential amplifier 30, which generates amplified signals proportional to differences in input signals at both ends provided from the signal providing unit 20, guidance voice for a CPR performing method, guidance voice related to whether CPR is being performed and whether CPR is being normally performed, and a simplified guidance voice and a detailed guidance voice related to whether CPR is being normally performed (S51).

The control unit 100 determines whether CPR is being performed and whether CPR is being normally performed, based on the speed of a plurality of amplified signals generated by the differential amplifier 30 (S52).

When an amplified signal generated by the differential amplifier 30 is received, the control unit 100 outputs guidance voice for a CPR performing method (S53).

When the control unit 100 determines, based on the speed of a plurality of amplified signals generated by the differential amplifier 30, that CPR is not being performed, it outputs the corresponding guidance voice (S54).

When the speed of a plurality of amplified signals generated by the differential amplifier 30 is within the normal speed range, the control unit 100 outputs the simplified guidance voice (S55).

When the speed of a plurality of amplified signals generated by the differential amplifier 30 deviates from the normal speed range, the control unit 100 outputs the detailed guidance voice (S56).

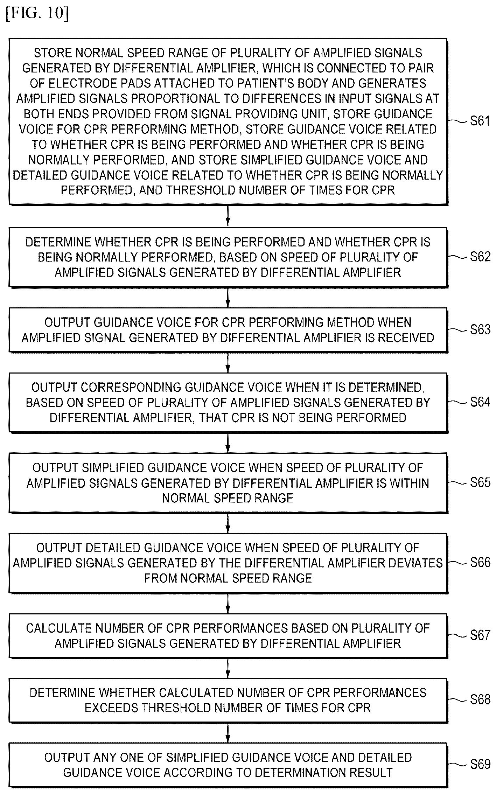

FIG. 10 is a flowchart of a fifth exemplary embodiment of a guidance method through detection of cardiopulmonary resuscitation (CPR) performance.

The control unit 100 is connected to a pair of electrode pads 11, 12 attached to the patient's body and stores guidance voice related to a normal speed range of a plurality of amplified signals generated by the differential amplifier 30, which generates amplified signals proportional to differences in input signals at both ends provided from the signal providing unit 20, guidance voice for a CPR performing method, guidance voice related to whether CPR is being performed and whether CPR is being normally performed, a simplified guidance voice and a detailed guidance voice related to whether CPR is being normally performed, and a threshold number of times for CPR (S61).

The control unit 100 determines whether CPR is being performed and whether CPR is being normally performed, based on the speed of a plurality of amplified signals generated by the differential amplifier 30 (S62).

When an amplified signal generated by the differential amplifier 30 is received, the control unit 100 outputs guidance voice for a CPR performing method (S63).

When the control unit 100 determines, based on the speed of a plurality of amplified signals generated by the differential amplifier 30, that CPR is not being performed, it outputs the corresponding guidance voice (S64).

When the speed of a plurality of amplified signals generated by the differential amplifier 30 is within the normal speed range, the control unit 100 outputs the simplified guidance voice (S65).

When the speed of a plurality of amplified signals generated by the differential amplifier 30 deviates from the normal speed range, the control unit 100 outputs the detailed guidance voice (S66).

The control unit 100 calculates the number of CPR performances based on a plurality of amplified signals generated by the differential amplifier 30 (S67).

The control unit 100 determines whether the calculated number of CPR performances exceeds a threshold number of times (S68).

The control unit 100 outputs any one of the simplified guidance voice and the detailed guidance voice according to the determination result (S69).

Here, referring to FIGS. 1 to 3, a measuring method through changes in thoracic impedance will be described.

A carrier signal in a range of 40 to 80 KHz is transmitted to both ends of a pair of electrode pads 11, 12, and an amplified signal proportional to the difference in input signals at both ends is generated through the differential amplifier 30, and this signal is converted into a DC signal through the rectifier 40.

When CPR is performed, the value of the DC signal varies according to a change in thoracic impedance between both ends of the pair of electrode pads 11, 12, and based on this change, it is possible to recognize whether CPR is being performed and to confirm the chest compression speed during CPR.

An output signal of the filter for CPR is illustrated in FIG. 2.

Criteria for determining whether CPR is normally performed will be described.

When no chest compression is performed within the chest compression monitoring setting time of the AED (setting range: 1 to 60 seconds) from the point of CPR operation, a detailed guidance voice is output, and when chest compression is performed, the compression speed is checked to evaluate whether it is performed at a rate of 100 to 120 times per minute. When it is performed within the range of 110 ± 20% per minute (this criterion may be changed at any time according to CPR guidelines, a relevant country, or customer request), a simplified guidance voice is output. When the speed deviates from the range of 110 ± 20% per minute, a detailed guidance voice is output.

The condition for detailed and simplified guidance is that when no CPR activity (chest compression) is performed within the set monitoring time, a detailed guidance voice is output, when the chest compression speed deviates from the set speed range, a detailed guidance voice is output; and when the chest compression speed is within the set speed range, a simplified guidance voice is output.

When the detailed guidance voice and the simplified guidance voice are determined, the guidance voice is provided accordingly, and a beep sound is generated according to the chest compression speed (100 to 120 times per minute) set by the metronome function.

When the rescuer performs chest compressions according to the metronome guidance, an average speed value is calculated as (T1 + T2 + ... + Tn)/Tn from the input signal of the chest compression speed. When this average speed value is faster than 120 times per minute, voice guidance coaching such as "Compress more slowly" is output, and when it is slower than 100 times per minute, voice guidance coaching such as "Compress more quickly" is output.

The chest compression speed may be checked in real time through the AED screen. Since coaching for chest compression speed in an emergency situation must be conducted concisely, voice guidance may be provided in simple expressions such as "faster" or "slower." When CPR feedback is provided using defibrillation pads, compression depth is not determined.

Possible modified exemplary embodiments other than the above exemplary embodiments will be described.

The guidance device through detection of cardiopulmonary resuscitation (CPR) performance may further include a camera for photographing the patient. When a pair of electrode pads is attached to the patient and the camera is disposed facing the patient, a signal is generated and provided to the pair of electrode pads from the signal providing unit, and photographing begins. The camera may photograph the rescuer performing CPR, and the control unit may store the photographed image.

The guidance device may further include a display unit for displaying an image of CPR, and the control unit stores normal chest compression postures of a plurality of rescuers performing CPR, determines whether the chest compression posture of a current rescuer performing CPR is equal to or greater than a preset similarity compared to at least any one of the plurality of normal chest compression postures, and, when it is not equal to or greater than the preset similarity, may display an image of the normal chest compression posture so that the rescuer may observe and follow it.

The control unit specifies a chest compression area from the photographed image of the patient, and when it determines that the chest compression area of the current rescuer performing CPR deviates from the specified chest compression area, a guidance voice notifying the specified chest compression area may be output. Here, the control unit may display the specified chest compression area on the display unit so that the rescuer may perform chest compressions accurately.

According to the guidance device through detection of cardiopulmonary resuscitation (CPR) performance 1 and the guidance method through detection of cardiopulmonary resuscitation (CPR) performance, when a pair of electrode pads is attached to the patient's chest area, it is possible to determine whether CPR is being performed based on amplitude differences of a plurality of amplified signals generated by the differential amplifier, and then to determine whether CPR is being normally performed according to whether the speed of the amplified signals of the differential amplifier is within the normal speed range, thereby enabling corresponding voice guidance to be output.

In addition, since instructions for CPR are provided to a non-professional rescuer, even a non-professional rescuer who is not a professional rescuer may easily perform CPR.

In addition, when there is no difference in input signals from the pair of electrode pads, it is determined that CPR is not being performed, and thus, the voice guidance regarding CPR performance and the CPR performing method may be output.

In addition, when CPR is normally performed, a simplified guidance voice of a beep sound corresponding to the set chest compression speed (100 to 120 times per minute) is output, and when CPR is abnormally performed, a detailed guidance voice regarding whether the chest compression speed is too slow or too fast may be output.

In addition, when the number of CPR performances does not exceed the threshold number of times, a simplified guidance voice of a beep sound corresponding to the set chest compression speed (100 to 120 times per minute) is output so that chest compressions may continue, and when the number of CPR performances exceeds the threshold number of times, a detailed guidance voice instructing to stop chest compressions may be output.

National (Korean) R&D projects supporting the present invention:
[Project ID] 1535000300
[Project No.] 202106862
[Ministry in charge] Korea Coast Guard
[Research management (specialist) agency] Korea Institute of Marine Science & Technology Promotion (KIMST)
[Research project name] R&D Project for Developing Search and Rescue Technology to Secure the Golden Hour
[Research title] Development of an Intelligent Portable On-Site Emergency Medical Kit to Improve the Survival Rate of Marine Emergency Patients
[Research institution name] CU Medical Systems, Inc
[Research period] April 30, 2021, to December 31, 2023

## Claims

1. A guidance device through detection of cardiopulmonary resuscitation (CPR) performance, comprising:
a pair of electrode pads configured to be attached to a patient's body;
a signal providing unit configured to provide a signal to the pair of electrode pads;
a differential amplifier connected to the pair of electrode pads and configured to generate an amplified signal proportional to a difference between input signals at both ends provided from the signal providing unit; and
a control unit configured to store a normal speed range for a plurality of amplified signals generated from the differential amplifier, and determine whether CPR is being performed and whether CPR is performed normally based on the plurality of amplified signals generated from the differential amplifier and a speed of the plurality of amplified signals generated from the differential amplifier.

2. The guidance device through detection of CPR performance according to claim 1, further comprising:
a speaker configured to output guidance voice for CPR,
wherein the control unit is configured to store the guidance voice regarding a CPR performing method and control the speaker so that the guidance voice regarding the CPR performing method is output when the amplified signal generated from the differential amplifier is received.

3. The guidance device through detection of CPR performance according to claim 2, wherein the control unit is configured to store guidance voice regarding whether CPR is being performed and whether CPR is performed normally, and control the speaker so that the guidance voice is output when it is determined, based on the plurality of amplified signals generated from the differential amplifier, that CPR is not being performed.

4. The guidance device through detection of CPR performance according to claim 3, wherein the control unit is configured to store a simplified guidance voice and a detailed guidance voice regarding whether CPR is performed normally, and control the speaker so that the simplified guidance voice is output when the speed of the plurality of amplified signals generated from the differential amplifier is within the normal speed range, and the detailed guidance voice is output when the speed of the plurality of amplified signals generated from the differential amplifier deviates from the normal speed range.

5. The guidance device through detection of CPR performance according to claim 4, wherein the control unit is configured to store a threshold number of times for CPR, calculate the number of performances of CPR based on the plurality of amplified signals generated from the differential amplifier, determine whether the calculated number of performances exceeds the threshold number of times, and control the speaker so that any one of the simplified guidance voice and the detailed guidance voice is output according to the determination result.

6. The guidance device through detection of CPR performance according to claim 1, wherein the control unit is configured to monitor, in real time, a state and performing accuracy of a rescuer during CPR performance, and output the detailed guidance voice step by step according to basic stages, intermediate processes, and special situations of CPR.

7. The guidance device through detection of CPR performance according to claim 1, wherein the control unit is configured to provide the detailed guidance voice to configured provide basic instructions on a chest compression position, compression depth, and compression rate in a basic stage of CPR, evaluate in real time the compression depth and rate in an intermediate process and provide feedback when they deviate from a normal range, and provide immediate instructions regarding fatigue of the rescuer, inaccuracy of hand position, or inconsistency of compression rhythm in a special situation.

8. The guidance device through detection of CPR performance according to claim 7, wherein the control unit is configured to attach a pressure sensor to a chest compression area of CPR to measure, in real time, pressure generated during CPR, calculate the compression depth and rate based on data collected through the pressure sensor, and convert the calculated result into voice and visual feedback.

9. The guidance device through detection of CPR performance according to claim 8, wherein the pressure sensor is configured to be one selected from a piezoresistive pressure sensor, a piezoelectric sensor, or a capacitive pressure sensor, and the control unit is configured to determine whether CPR is being performed and whether CPR is performed normally based on the pressure data detected from the pressure sensor.

10. The guidance device through detection of CPR performance according to claim 9, wherein the control unit is configured to provide immediate guidance to the rescuer with voice and visual guidance through the speaker and a display unit when the compression depth is insufficient or excessive, or when the compression rate deviates from a normal range, based on the data collected from the pressure sensor.

11. A guidance method through detection of cardiopulmonary resuscitation (CPR) performance, comprising:
storing a normal speed range for a plurality of amplified signals generated from a differential amplifier that is connected to a pair of electrode pads attached to a patient's body and configured to generate the amplified signals proportional to a difference between input signals at both ends provided from a signal providing unit; and
determining, based on a speed of the plurality of amplified signals generated from the differential amplifier, whether CPR is being performed and whether CPR is performed normally.

12. The guidance method through detection of cardiopulmonary resuscitation performance according to claim 11, further comprising:
storing guidance voice regarding a CPR performing method; and
outputting the guidance voice regarding the CPR performing method when the amplified signal generated from the differential amplifier is received.

13. The guidance method through detection of CPR performance according to claim 12, further comprising:
storing the guidance voice regarding whether CPR is being performed and whether CPR is performed normally; and
outputting the guidance voice when it is determined, based on the speed of the plurality of amplified signals generated from the differential amplifier, that CPR is not being performed.

14. The guidance method through detection of CPR performance according to claim 13, further comprising:
storing a simplified guidance voice and a detailed guidance voice regarding whether CPR is performed normally;
outputting the simplified guidance voice when the speed of the plurality of amplified signals generated from the differential amplifier is within the normal speed range; and
outputting the detailed guidance voice when the speed of the plurality of amplified signals generated from the differential amplifier deviates from the normal speed range.

15. The guidance method through detection of CPR performance according to claim 14, further comprising:
storing a threshold number of times for CPR;
calculating the number of performances of CPR based on the plurality of amplified signals generated from the differential amplifier;
determining whether the calculated number of performances exceeds the threshold number of times; and
outputting any one of the simplified guidance voice and the detailed guidance voice according to the determination result.
